# EUROPEAN PATENT SPECIFICATION

(11) **EP 3 137 207 B1**
(45) Date of publication and mention of the grant of the patent: **06.02.2019**
(21) Application number: 15718490.4
(22) Date of filing: 27.04.2015
(51) Int. Cl.: B01J 13/16

(54) **ANIONIC POLYVINYL ALCOHOL COPOLYMER AS PROTECTIVE COLLOID FOR PESTICIDAL POLYUREA MICROCAPSULES**
ANIONISCHES POLYVINYL-ALKOHOL-COPOLYMER ALS SCHUTZKOLLOID FÜR PESTIZID-POLYHARNSTOFF-MIKROKAPSELN
COPOLYMÈRES D'ALCOOL POLYVINYLIQUE ANIONIQUES EN TANT QUE COLLOÏDE PROTECTEUR DANS DES MICROCAPSULES DE POLYURÉE PESTICIDES

(30) Priority: 29.04.2014 EP 14166360; 30.03.2015 US 201562139819 P
(43) Date of publication of application: 08.03.2017
(73) Proprietor: BASF SE, 67056 Ludwigshafen am Rhein (DE)
(72) Inventor: BURAKOWSKA-MEISE, Ewelina, 64686 Reichenbach (Lautertal) (DE); MECFEL-MARCZEWSKI, Joanna, 67117 Limburgerhof (DE); BRATZ, Matthias, 67133 Maxdorf (DE); DENUELL, Wolfgang, 68163 Mannheim (DE); BOWE, Steven, Apex, NC 27523 (US); REPAGE, Ronald, 69190 Walldorf (DE); FRIHAUF, John, Lincoln, NE 68523 (US)
(74) Representative: BASF IP Association
(86) International application number: PCT/EP2015/059001
(87) International publication number: WO 2015/165834

(56) References cited:
- EP-A1- 2 399 667
- WO-A1-98/03065
- US-A- 5 342 556
- DATABASE WPI Week 199831 1998 Thomson Scientific, London, GB; AN 1998-357509 XP002733594, & JP H10 139818 A (KURARAY CO LTD) 26 May 1998 (1998-05-26)

## Description

The present invention relates to a process for producing microcapsules which contain a shell and a core of a liquid water-insoluble material, where
(a) a premix (I) is prepared from water and a protective colloid;
(b) a further premix (II) is prepared from the water-insoluble material and at least bifunctional isocyanate (A) or a mixture of two or more different isocyanates containing (A);
(c) the two premixes (I) and (II) are mixed together until an emulsion is formed;
(d) at least a bifunctional amine is then poured into the emulsion from step (c); and
(e) the emulsion is then heated until the microcapsules are formed, and
where the liquid water-insoluble material comprises a pesticide, where the protective colloid is a polyvinyl alcohol copolymer having hydrolysis degrees from 60 to 99.9 %, and where the polyvinyl alcohol copolymer contains comonomers with anionic groups and wherein the isocyanate (A) is selected from alicyclic or aliphatic isocyanates.

Further subject matter are microcapsules obtainable by said process. The present invention also relates to a method of controlling phytopathogenic fungi and/or undesired plant growth and/or undesired insect or mite attack and/or for regulating the growth of plants, wherein the microcapsules obtainable by said process are allowed to act on the respective pests, their environment or the crop plants to be protected from the respective pest, on the soil and/or on undesired plants and/or on the crop plants and/or on their environment.

Microcapsules are usually spherical objects which consist of a core and a wall material surrounding the core, wherein the core is a solid, liquid or gaseous substance which is surrounded by the solid (generally polymeric) wall material. They may be solid, i.e. consist of a single material. Microcapsules may have a diameter from 1 to 1000 µm, on average.

A multitude of shell materials is known for producing the wall of microcapsules. The shell can consist either of natural, semisynthetic or synthetic materials. Natural shell materials are, for example, gum arabic, agar agar, agarose, maltodextrins, alginic acid or its salts, e.g. sodium alginate or calcium alginate, fats and fatty acids, cetyl alcohol, collagen, chitosan, lecithins, gelatin, albumin, shellac, polysaccharides, such as starch or dextran, polypeptides, protein hydrolyzates, sucrose and waxes. Semisynthetic shell materials are inter alia chemically modified celluloses, in particular cellulose esters and cellulose ethers, e.g. cellulose acetate, ethyl cellulose, hydroxypropylcellulose, hydroxypropylmethylcellulose and carboxymethylcellulose, and also starch derivatives, in particular starch ethers and starch esters. Synthetic shell materials are, for example, polymers such as polyacrylates, polyamides, polyvinyl alcohol or polyurea.

Depending on the type of shell material and the production process, microcapsules are formed in each case with different properties, such as diameter, size distribution and physical and/or chemical properties.

Polyurea core-shell microcapsules obtained by reaction of two diisocyanates and a polyamine are well known in the art.

WO98/03065 A1 discloses a method for producing microcapsules containing a shell and a liquid water insoluble pesticidal core, the process comprises the step of preparing an aqueous anionic polyvinyl alcohol colloid and mixing it with aromatic polyisocyanate and the pesticide chlorpyrifos.

US 5342556 A discloses a method to produce gamma-hexachlorocyclohexane in chloroparaffin in a microcapsule shell of hexamethylene diisocyanate biuret crosslinked with ethylene diamine.

To provide microcapsules with tailored properties novel production processes need to be developed. Especially microcapsules produced to encapsulate water-insoluble ingredients like oils need to have an optimized, enhanced stability against leaking-out of the oil from the capsules into the external phase, particularly in surfactant-based formulations. Moreover, an aqueous dispersion of the microcapsules needs to be stable against separation over a long period of time.

There is an ongoing need to provide capsules for the formulation of pesticides, where the capsule slowly releases the pesticide over a prolonged period in order to decrease losses by rain wash or detrimental effects such as phytotoxicity.

The problem was solved by a process for producing microcapsules which contain a shell and a core of a liquid water-insoluble material, where
(a) a premix (I) is prepared from water and a protective colloid;
(b) a further premix (II) is prepared from the water-insoluble material and at least bifunctional isocyanate (A) or a mixture of two or more different isocyanates containing (A);
(c) the two premixes (I) and (II) are mixed together until an emulsion is formed;
(d) at least a bifunctional amine is then poured into the emulsion from step (c); and
(e) the emulsion is then heated (e.g. up to at least 50°C) until the microcapsules are formed, and
where the liquid water-insoluble material comprises a pesticide,
where the protective colloid is a polyvinyl alcohol copolymer having hydrolysis degrees from 60 to 99.9 % (preferably from 85 to 99.9 %), and where the copolymer contains comonomers with anionic groups and wherein the isocyanate (A) is selected from alicyclic or aliphatic isocyanates. In steps (a), (b), (c), (d), and (e) further auxiliaries may be present and/or may be added.

Also defined are microcapsules obtainable by the process according to the invention.

According to the invention microcapsules with determined sizes and/or size distribution can be produced in a targeted manner. Moreover, it is possible to produce relatively small microcapsules with diameters from 5 to 30 µm. Advantageously, capsules with greater enhanced leakage stability against leakage in surfactant-based formulations are obtainable, which show a better performance against separation over a long period of time.

According to the invention a mixture of bifunctional isocayanates (A) and isocyanates (B) can be added in one step (e.g. step (b)) or can be added separately from each other.

According to one embodiment of the invention the bifunctional isocyanates (A) are dissolved alone or in a mixture with a further isocyanate (B) in the water-insoluble liquid (also termed "water-insoluble material") which later forms the core of the microcapsules; the premixes (I) and (II) are mixed together until an emulsion is formed and then the amine components are added and the mixture is heated until the capsules are formed.

According to a second embodiment of the invention the bifunctional isocyanates (A) are dissolved alone in the water-insoluble material which later forms the core of the microcapsules; the premixes (I) and (II) are mixed together until an emulsion is formed and then the further isocyanate (B) is added before the amine components are added and the mixture is heated until the capsules are formed.

The temperature for the reaction of the isocyanates with the amine components (e.g. in step (e)) may be at least 50°C, better 60°C, preferably 75 to 90°C and in particular 85 to 90°C, in order to ensure sufficiently rapid reaction progress.

Here, it may be preferred to increase the temperature in stages (e.g. in each case by 10°C) until then, following completion of the reaction, the dispersion is cooled down to room temperature (21°C).

The reaction time typically depends on the reaction amount and temperature used. Usually, microcapsule formation is established between ca. 60 minutes to 6 h or up to 8 h at the temperatures defined above.

According to the present teaching, the addition of the amine also preferably takes place with the input of energy, e.g. by using a stirring apparatus.

In order to form an emulsion in the present process, the respective mixtures are usually emulsified by processes known to the person skilled in the art, e.g. by introducing energy into the mixture through stirring using a suitable stirrer until the mixture emulsifies. The pH is preferably adjusted using aqueous bases, preference being given to using sodium hydroxide solution (e.g. 5% strength by weight). It may be advantageous to adjust the pH of premix (I) from 3 to 12, preferably between 4 to 10, and in particular in the range from 5 to 10.

### Microcapsules

The microcapsules are preferably obainable by the process according to the invention.

Within the context of the present teaching, the microcapsules may have a shell made by a polyaddition between at least bifunctional isocyanates with amines, preferably with polyamines, which leads to polyurea derivatives.

The microcapsules are present in the form of aqueous dispersions, the weight fraction of these dispersions in the microcapsules being favored between 5 and 50% by weight, preferably between 15 to 40% per weight and preferably 20 to 40% by weight. The microcapsules usually have an average **diameter** in the range from 1 to 500 µm and preferably from 3 to 50 µm or from 5 to 30 µm. The particle size determinations specified are carried out by means of static
laser diffraction. The d 50 and d 90 values may be based on the volume distribution of the particles.

Microcapsules disclosed but not claimed contain an oil as the water-insoluble material. The fraction of this oil can vary in the range from 10 to 95% by weight, based on the weight of the microcapsules, where fractions from 70 to 90% by weight may be advantageous. Result of the process are microcapsules obtainable which typical core-shell ratios (w/w) from 20:1 to 1:10, preferably from 5:1 to 2:1 and in particular from 4:1 to 3:1.

The microcapsules which are obtainable by the present processes are preferably free from formaldehyde.

### Protective colloid

It is state of the art to use protective colloids like polyvinyl alcohols during the reaction between isocyanates and amines.

Polyvinyl alcohol (= PVA) corresponds typically in general according to formula 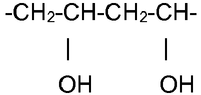 with low amounts (up to 2%) of the formula structure

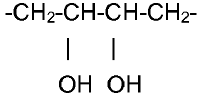

In order to get the benefits of the invention it is essential to use special protective colloids of polyvinyl alcohol. It was found that the microcapsules have superior properties when **polyvinyl alcohol copolymers** are used having a hydrolysis degree 60 to 99.9% (preferably from 85 to 99.9 %).

According to the invention the term "polyvinyl alcohol copolymer" means a polymer of vinyl alcohol/vinyl acetate with comonomers.

It is known that polyvinyl alcohol is produced by hydrolysis (deacetylation) of polyvinyl acetate, whereby the ester groups of polyvinyl acetate are hydrolysed into hydroxyl groups, thus forming polyvinyl alcohol.

The degree of hydrolysis is a criteria of how many groups are converted into hydroxyl groups. The term "polyvinyl alcohol" in connection with a given degree of hydrolysis means therefore, in fact, a vinyl polymer containing ester and hydroxylgroups.

According to the invention polyvinyl alcohol copolymers with degrees of hydrolysis from 85 to 99.9%, especially between 85 to 95% may be used. In another form polyvinyl alcohol copolymers with degrees of hydrolysis from 60 to 99.9%, preferably from 70 to 98%, more preferably from 75 to 97 %, and in particular from 85 to 96 % may be used.

The degree of hydrolysis of polyvinyl alcohol may be determined according to DIN 53401.

The polyvinyl alcohol polymers according to the invention contain additional comonomers, i.e. other comonomers are polymerized together with vinylester in a first step, followed by the hydrolysis of the ester groups to form the copolymer of polyvinyl alcohol in a second step.

It is state of the art to prepare copolymers of polyvinyl alcohol by a radical polymerization reaction between the vinyl acetate and comonomers.

According to the invention polyvinyl alcohol copolymers preferably contain unsaturated hydrocarbons as comonomers. These unsaturated hydrocarbons are optionally modified with functional non-charged and/or charged groups.

In particular the following comonomers are suitable: unsaturated hydrocarbons having anionic groups like carboxyl- and/or sulfonic acid groups. In another form the polyvinyl alcohol copolymer contains comonomers with anionic groups selected from carboxyl- and/or sulfonic acid groups. In another form the polyvinyl alcohol copolymer contains 0.1 to 30 mol% (preferably 0.3 to 20 mol%, more preferably 0.5 to 10 mol%) of the comonomers with anionic groups.

It is preferred according to the invention to use copolymers of polyvinyl alcohol with hydrolysis degrees from 85 to 99.9, preferred 85 % to 95% and containing 0.1 to 30 mol% (preferably 0.3 to 20 mol%, more preferably 0.5 to 10 mol%) comonomers with anionic groups like carboxyl- and/or sulfonic acid groups, wherein mol% is based on polymerization mixture vinyl acetate/comonomer.

In another form it is preferred according to the invention to use copolymers of polyvinyl alcohol with hydrolysis degrees from 60 to 99.9, preferred 70 % to 98%, and containing 0.1 to 30 mol% (preferably 0.3 to 20 mol%, more preferably 0.5 to 10 mol%) comonomers with anionic groups like carboxyl- and/or sulfonic acid groups, wherein mol% is based on polymerization mixture vinyl acetate/comonomer.

The polyvinyl alcohol copolymer may have a viscosity from 1 to 100 mPas, preferably from 1.5 to 70 mPas, more preferably from 2 to 50 mPas. The viscosity may be determined according to Brookfield at 4% in water at 20 °C.

Suitable copolymers of polyvinyl alcohol and anionic comonomers, are described in EP 2426172, EP 2648211, GB1438449 and EP0291198.

Following protective colloids are particular suitable for the production of microcapsules according to the invention: Anionic polyvinyl alcohol copolymers with the hydrolysis degree > 80% - preferably 85.0%-99.5% and the viscosity 2 mPas-70 mPas (DP 100-6000). In another form anionic polyvinyl alcohol copolymers with the hydrolysis degree from 60 to 99.9 %, preferably from 85.0%-99.5%, and the viscosity from 2 mPas to 70 mPas (DP 100-6000). Examples of such type of colloids are: K-Polymer 25-88KL from Kuraray (viscosity 20-30 mPas, hydrolysis 85.0-90.0%), Gohsenal T-350 from Nippon Gohsei (viscosity 27-33 mPas, hydrolysis 93.0-95.0%), Gohseran L-3266 from Nippon Gohsei (viscosity 2.3-2.7 mPas, hydrolysis 86.5-89.0%).

In general, the protective colloids (e.g. the polyvinyl alcohol copolymer) are used with amounts from 0.1 to 20% by weight, but preferably in the range from 1 to 10% by weight and in particular from 1.5 to 5% by weight, based on the weight of the microcapsules. The weight of the microcapsules is usually based on the total sum of the shell and the core materials, e.g. all isocyanates, all bifunctional amines, all water-insuluble materials, and the polyvinyl alcohol copolymer.

Combinations of two or more different protective colloids may also be beneficial.

### Isocyanates

Isocyanates are N-substituted organic derivatives (R-N=C=O) of isocyanic acid (HNCO) tautomeric in the free state with cyanic acid. Organic isocyanates are compounds in which the isocyanate group (-N=C=O) is bonded to an organic radical. Polyfunctional isocyanates are those compounds with two or more isocyanate groups in the molecule.

The isocyanates are as defined in claim 1. Polyfunctional isocyanates are used as (A) provided they have at least two reactive isocyanate groups. The alicyclic isocyanate is more preferred.

The suitable **polyfunctional isocyanates (A)** preferably contain on average 2 to at most 4 NCO groups. Preference is given to using diisocyanates, i.e. esters of isocyanic acid with the general structure O=C=N-R-N=C=O, where R' here is aliphatic, alicyclic or aromatic radicals.

Suitable isocyanates (A) are the isocyanates of the following list that are aliphatic or alicyclic: for example, 1,5-naphthylene diisocyanate, 4,4'-diphenylmethane diisocyanate (MOI), hydrogenated MDI (H12MDI), xylylene diisocyanate (XDI), tetramethylxylol diisocyanate (TMXDI), 4,4'-diphenyldimethylmethane diisocyanate, di- and tetraalkyldiphenyl-methane diisocyanate, 4,4'-dibenzyl diisocyanate, 1,3-phenylene diisocyanate, 1,4-phenylene diisocyanate, the isomers of tolylene diisocyanate (TDI), optionally in a mixture, 1-methyl-2,4-diisocyanatocyclohexane, 1,6-diisocyanato-2,2,4-trimethylhexane, 1,6-diisocyanato-2,4,4-trimethylhexane, 1-isocyanatomethyl-3-isocyanato-1,5,5-trimethylcyclohexane, chlorinated and brominated diisocyanates, phosphorus-containing diisocyanates, 4,4'-diisocyanatophenylperfluoroethane, tetramethoxybutane 1,4-diisocyanate, butane 1,4-diisocyanate, hexane 1,6-diisocyanate (HDI), dicyclohexylmethane diisocyanate, cyclohexane 1,4-diisocyanate, ethylene diisocyanate, phthalic acid bisisocyanatoethyl ester, also polyisocyanates with reactive halogen atoms, such as 1-chloromethylphenyl 2,4-diisocyanate, 1-bromomethylphenyl 2,6-diisocyanate, 3,3-bischloromethyl ether 4,4'-diphenyldiisocyanate. Sulfur-containing polyisocyanates are obtained, for example, by reacting 2 mol of hexamethylene diisocyanate with 1 mol of thiodiglycol or dihydroxydihexyl sulfide. Further suitable diisocyanates are trimethylhexamethylene diisocyanate, 1,4-diisocyanatobutane, 1,2-diisocyanatododecane and dimer fatty acid diisocyanate.

Suitable isocyanates of type (A) are at least bifunctional compounds (i.e. compounds containing at least two isocyanate groups -N=C=O).Typical representatives may be hexamethylene diisocyanate (HDI), or derivatives thereof, e.g. HDI biuret (commercially available e.g. as Desmodur N3200), HDI trimers (commercially available as Desmodur N3300) or else dicyclohexylmethane diisocyanates (commercially available as Desmodur W). Toluene 2,4-diisocyanate or diphenylmethane diisocyanate are also disclosed as isocyanates.

Preferred according to the invention are isocyanates of type (A), selected from the group consisting of hexane 1,6-diisocyanate, hexane 1,6-diisocyanate biuret or oligomers of hexane 1,6-diisocyanate, in particular trimers thereof or dicyclohexanemethylene diisocyanate. In another form the isocyanate (A) is hexane 1,6-diisocyanate, hexane 1,6-diisocyanate biuret, dicyclohexylmethane diisocyanates, or oligomers of hexane 1,6-diisocyanate.

One essential feature of the present process is the use of two structurally different isocyanates (A) and (B).

The second isocyanate of type (B) is structurally different from the isocyanate of type (A) and specifically the isocyanate of type (B) could either be an anionically modified isocyanate or a polyethylene oxide-containing isocyanate (or any desired mixtures of these two isocyanate types).

The **anionically modified isocyanates** are known per se. Preferably, these isocyanates of type (B) contain at least two isocyanate groups in the molecule. One or more sulfonic acid radicals are preferably present as anionic groups. Preferably, isocyanates of type (B) are selected which are oligomers, in particular trimers, of hexane 1,6-diisocyanate (HDI). Commercial products of these anionically modified isocyanates are known, for example, under the brand Bayhydur (Bayer), e.g. Bayhydur XP.

Polyethylene oxide-containing isocyanates (with at least two isocyanate groups) are also known and are described, e.g. in US 5,342,556. Some of these isocyanates are self-emulsifying in water, which may be advantageous within the context of the present process since it may be possible to dispense with a separate emulsifying step.

The weight ratio of the two isocyanates (A) and (B) is adjusted preferably in the range from 10:1 to 1:10, more preferably in the range from 5:1 to 1:5 and in particular in the range from 3:1 to 1:1.

It is also possible to use mixtures of different isocyanates of types (A) and (B). Besides the isocyanates (A) and (B), further isocyanates can also additionally be used in the process according to the invention.

Preferably, however, a mixture of isocyanate (A) and an anionically modified isocyanate (B) is used, wherein the anionically modified diisocyanates (B) are selected from the group which contains at least one sulfonic acid group, preferably an aminosulfonic acid group, in the present process.

In another form in general it is preferred to use 0.5 - 12 wt.%, in particular between 4 - 8 wt.%, total sum of isocyanates (A) and (B) based on the total weight of the compounds used in the process.

### Amines

At least bifunctional amines, but preferably polyethyleneimines (PEI), are used as further component in the process according to the invention. Polyethyleneimines are generally polymers in the main chains of which there are NH groups which are separated from one another in each case by two methylene groups:

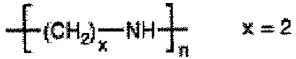

Polyethyleneimines belong to the polyelectrolytes and the complexing polymers. Short-chain, linear polyethyleneimines with a correspondingly high fraction of primary amino groups, i.e. products of the general formula H₂N ⁅CH₂-CH₂-NH⁆*ₙ*H (*n* = 2: diethylenetriamine; *n* = 3; triethylenetetramine; *n* = 4: tetraethylenepentamine) are sometimes called polyethyleneamines or polyalkylenepolyamines.

In the processes according to the invention, polyethyleneimines with a molecular weight of at least 500 g/mol, preferably from 600 to 30 000 or 650 to 25 000 g/mol and in particular from 700 to 5000 g/mol or 850 to 2500 g/mol, are preferably used. In general it is preferred to use 0.3 - 10 wt.%, in particular between 0.5 - 5 wt.%, polyethyleneimines. In another form In general it is preferred to use 0.3 - 10 wt.%, in particular between 0.5 - 5 wt.%, polyethyleneimines based on the total weight of the compounds used in the process.

In one form microcapsules are obtained with a diameter from 1 to 30 µm comprising a liquid core of a water-insoluble material, and a shell of a reaction product of an at least bifunctional isocyanate (A) or a mixture of two or more different isocyanates containing (A) and an at least bifunctional amine in presence of polyvinyl alcohol copolymer with hydrolysis degrees above 85 to 99.9 % as a protective colloid.

### Water-insoluble material

The microcapsules produced using the process described above contain in the interior a pesticide that is preferably water-insoluble and liquid at 21°C (i.e. at 21°C, a maximum of 10 g of the material can be dissolved in 1 l of water). This includes all types of hydrophobic water-insoluble liquids, and any blends thereof. Excluded are usually any fragrances or perfumes as such materials.

Also disclosed but not claimed is a water-insoluble materials also known as an oil. These oils must be able, preferably without auxiliaries, to dissolve the isocyanates in order to be able to use them in the present process. Should an oil not ensure adequate solubility of the isocyanates, there is the option of overcoming this disadvantage by using suitable solubility promoters.

Besides the aforementioned oils, the microcapsules can also have further, optionally liquid or solid, ingredients which are dissolved, dispersed or emulsified in the oil in the microcapsules. The phrase "oil" in the context of the present invention encompasses all kinds of oil bodies or oil components, in particular vegetable oils like e.g. rape seed oil, sunflower oil, soy oil, olive oil and the like, modified vegetable oils e.g. alkoxylated sunflower or soy oil, synthetic (tri)glycerides like e.g. technical mixtures of mono, di and triglycerides of C6-C22 fatty acids, fatty acid alkyl esters e.g. methyl or ethyl esters of vegetable oils (Agnique® ME 18 RD-F, Agnique® ME 18 SD-F, Agnique® ME 12C-F, Agnique® ME1270, all products of Cognis GmbH, Germany) fatty acid alkyl esters based on said C6-C22 fatty acids, mineral oils and their mixtures. In one form the oil comprises preferably mineral oils.

Examples illustrating the nature of suitable hydrophobic carriers without limiting the invention to these examples are: Guerbet alcohols based on fatty alcohols having 6 to 18, preferably 8 to 10, carbon atoms, esters of linear C6-C22-fatty acids with linear or branched C6-C22-fatty alcohols or esters of branched C6-C 13-carboxylic acids with linear or branched C6-C 22-fatty alcohols, such as, for example, myristyl myristate, myristyl palmitate, myristyl stearate, myristyl isostearate, myristyl oleate, myristyl behenate, myristyl erucate, cetyl myristate, cetyl palmitate, cetyl stearate, cetyl isostearate, cetyl oleate, cetyl behenate, cetyl erucate, stearyl myristate, stearyl palmitate, stearyl stearate, stearyl isostearate, stearyl oleate, stearyl behenate, stearyl erucate, isostearyl myristate, isostearyl palmitate, isostearyl stearate, isostearyl isostearate, isostearyl oleate, isostearyl behenate, isostearyl oleate, oleyl myristate, oleyl palmitate, oleyl stearate, oleyl isostearate, oleyl oleate, oleyl behenate, oleyl erucate, behenyl myristate, behenyl palmitate, behenyl stearate, behenyl isostearate, behenyl oleate, behenyl behenate, behenyl erucate, erucyl myristate, erucyl palmitate, erucyl stearate, erucyl isostearate, erucyl oleate, erucyl behenate and erucyl erucate. Also suitable are esters of linear C6-C22-fatty acids with branched alcohols, in particular 2-ethylhexanol, esters of C18-C38- alkylhydroxy carboxylic acids with linear or branched C6-C 22-fatty alcohols, in particular Dioctyl Malate, esters of linear and/or branched fatty acids with polyhydric alcohols (such as, for example, propylene glycol, dimerdiol or trimertriol) and/or Guerbet alcohols, triglycerides based on C6-C10-fatty acids, liquid mono-/di-/triglyceride mixtures based on C6-C18-fatty acids, esters of C6-C22-fatty alcohols and/or Guerbet alcohols with aromatic carboxylic acids, in particular benzoic acid, esters of C2- C12-dicarboxylic acids with linear or branched alcohols having 1 to 22 carbon atoms or polyols having 2 to 10 carbon atoms and 2 to 6 hydroxyl groups, vegetable oils, branched primary alcohols, substituted cyclohexanes, linear and branched C6-C22-fatty alcohol carbonates, such as, for example, dicaprylyl carbonate (Cetiol® CC), Guerbet carbonates, based on fatty alcohols having 6 to 18, preferably 8 to 10, carbon atoms, esters of benzoic acid with linear and/or branched C6-C22-alcohols, linear or branched, symmetrical or asymmetrical dialkyl ethers having 6 to 22 carbon atoms per alkyl group, such as, for example, dicaprylyl ether, ring-opening products of epoxidized fatty acid esters with polyols, silicone oils (cyclomethicones, silicone methicone grades, etc.), aliphatic or naphthenic hydrocarbons, such as, for example, squalane, squalene or dialkylcyclohexanes, and/or mineral oils. In one form the oil comprises preferably aliphatic or naphthenic hydrocarbons, and/or mineral oils.

Preferred oils are, Guerbet alcohols based on fatty alcohols having 6 to 18, preferably 8 to 10, carbon atoms, esters of linear C6-C22-fatty acids with linear or branched C6-C22-fatty alcohols or esters of branched C6-C13-carboxylic acids with linear or branched C6-C22-fatty alcohols, such as e.g. myristyl myristate, myristyl palmitate, myristyl stearate, myristyl isostearate, myristyl oleate, myristyl behenate, myristyl erucate, cetyl myristate, cetyl palmitate, cetyl stearate, cetyl isostearate, cetyl oleate, cetyl behenate, cetyl erucate, stearyl myristate, stearyl palmitate, stearyl stearate, stearyl isostearate, stearyl oleate, stearyl behenate, stearyl erucate, isostearyl myristate, isostearyl palmitate, isostearyl stearate, isostearyl isostearate, isostearyl oleate, isostearyl behenate, isostearyl oleate, oleyl myristate, oleyl palmitate, oleyl stearate, oleyl isostearate, oleyl oleate, oleyl behenate, oleyl erucate, behenyl myristate, behenyl palmitate, behenyl stearate, behenyl isostearate, behenyl oleate, behenyl behenate, behenyl erucate, erucyl myristate, erucyl palmitate, erucyl stearate, erucyl isostearate, erucyl oleate, erucyl behenate and erucyl erucate.

Also preferred oils are esters of linear C6-C22-fatty acids with branched alcohols, in particular 2-ethylhexanol, esters of C18-C38-alkylhydroxycarboxylic acids with linear or branched C6-C22-fatty alcohols, linear or branched C6-C22-fatty alcohols, in particular dioctyl malates, esters of linear and/or branched fatty acids with polyhydric alcohols (such as e.g. propylene glycol, dimerdiol or trimertriol) and/or Guerbet alcohols, triglycerides based on C6-C10-fatty acids, liquid mono-/di-/triglyceride mixtures based on C6-C18-fatty acids, esters of C6-C22-fatty alcohols and/or Guerbet alcohols with aromatic carboxylic acids, in particular benzoic acid, esters of C2-C12-dicarboxylic acids with linear or branched alcohols having 1 to 22 carbon atoms or polyols having 2 to 10 carbon atoms and 2 to 6 hydroxyl groups, vegetable oils, branched primary alcohols, substituted cyclohexanes, linear and branched C6-C22-fatty alcohol carbonates, such as e.g. dicaprylyl carbonate (Cetiol™ CC), Guerbet carbonates based on fatty alcohols having 6 to 18, preferably 8 to 10, carbon atoms, esters of benzoic acid with linear and/or branched C6-C22-alcohols (e.g. Finsolv™ TN), linear or branched, symmetrical or asymmetrical dialkyl ethers having 6 to 22 carbon atoms per alkyl group, such as e.g. dicaprylyl ether (Cetiol™ OE), ring-opening products of epoxidized fatty acid esters with polyols, silicone oils (cyclomethicones, silicon methicone types etc.) and/or aliphatic or naphthenic hydrocarbons, such as e.g. squalane, squalene or dialkylcyclohexanes.

Furthermore, liquid linear and/or branched and/or saturated or unsaturated hydrocarbons or any desired mixtures thereof can be used as oils within the context of the present invention. These may be e.g. alkanes having 4 to 22, preferably 6 to 18, carbon atoms, or any desired mixtures thereof. Also of suitability are the unsaturated hydrocarbons having 4 to 22 carbon atoms, or unsaturated hydrocarbons of identical carbon number, and any desired mixtures of these hydrocarbons. Cyclic hydrocarbons and aromatics, e.g. toluene and mixtures thereof may also be oils within the context of the present invention. In another preferred form the oil comprises aromatics.Also suitable are silicone oils. Any desired mixtures of all of the specified core materials are also suitable.

It is also possible for other liquid, preferably water-insoluble materials, such as biocides to be used and be present in the microcapsules. Any desired mixtures of these further materials may also be present in the microcapsules. In cases where such material is not oil-soluble, additives may be used for dispersing or emulsifying it. Otherwise, many actives, as for example biocides or dyes often only available as blends with an oily solvent. Those compositions are also useful in the context of the present invention. In another preferred form the water-insoluble material comprises a pesticide blended with an oily solvent (also termed "oil" above). Most preferred is the use of biocides (in particular pesticides), in the microcapsules of the present invention.

In a more preferred form the water-insoluble material comprises a pesticide blended with an oily solvent selected from aliphatic and/or aromatic hydrocarbons.

### Biocides

A biocide is a chemical substance capable of killing different forms of living organisms used in fields such as medicine, agriculture, forestry, and mosquito control. Usually, biocides are divided into two sub-groups:
- pesticides, which includes fungicides, herbicides, insecticides, algicides, moluscicides, miticides and rodenticides, and
- antimicrobials, which includes germicides, antibiotics, antibacterials, antivirals, antifungals, antiprotozoals and antiparasites.

### Pesticides:

The U.S Environmental Protection Agency (EPA) defines a pesticide as "any substance or mixture of substances intended for preventing, destroying, repelling, or mitigating any pest". The skilled worker is familiar with such pesticides, which can be found, for example, in the Pesticide Manual, 16th Ed. (2013), The British Crop Protection Council, London. A pesticide may be a chemical substance or biological agent (such as a virus or bacteria) used against pests including insects, plant pathogens, weeds, mollusks, birds, mammals, fish, nematodes (roundworms) and microbes that compete with humans for food, destroy property, spread disease or are a nuisance. In the following examples, pesticides suitable for the agrochemical compositions according to the present invention are given:
Fungicides: A fungicide is one of three main methods of pest control - the chemical control of fungi in this case. Fungicides are chemical compounds used to prevent the spread of fungi in gardens and crops. Fungicides are also used to fight fungal infections. Fungicides can either be contact or systemic. A contact fungicide kills fungi when sprayed on its surface. A systemic fungicide has to be absorbed by the fungus before the fungus dies. Examples for suitable fungicides, according to the present invention, encompass the following species: (3-ethoxypropyl)mercury bromide, 2-methoxyethy¬mercury chloride, 2-phenylphenol, 8-hydroxyquinoline sulfate, 8-phenylmercurioxy¬quinoline, acibenzolar, acylamino acid fungicides, acypetacs, aldimorph, aliphatic nitrogen fungicides, allyl alcohol, amide fungicides, ampropylfos, anilazine, anilide fungicides, antibiotic fungicides, aromatic fungicides, aureofungin, azaconazole, azithiram, azoxystrobin, barium polysulfide, benalaxyl, benalaxyl-M, benodanil, benomyl, benquinox, bentaluron, benthiavalicarb, benzalkonium chloride, benzamacril, benzamide fungicides, benzamorf, benzanilide fungicides, benzimidazole fungicides, benzimidazole precursor fungicides, benzimidazolylcarbamate fungicides, benzohydroxamic acid, benzothiazole fungicides, bethoxazin, binapacryl, biphenyl, bitertanol, bithionol, blasticidin-S, Bordeaux mixture, boscalid, bridged diphenyl fungicides, bromuconazole, bupirimate, Burgundy mixture, buthiobate, butylamine, calcium polysulfide, captafol, captan, carbamate fungicides, carbamorph, carbanilate fungicides, carbendazim, carboxin, carpropamid, carvone, Cheshunt mixture, chinomethionat, chlobenthiazone, chloraniformethan, chloranil, chlorfenazole, chlorodinitronaphthalene, chloroneb, chloropicrin, chlorothalonil, chlorquinox, chlozolinate, ciclopirox, climbazole, clotrimazole, conazole fungicides, conazole fungicides (imidazoles), conazole fungicides (triazoles), copper(II) acetate, copper(II) carbonate, basic, copper fungicides, copper hydroxide, copper naphthenate, copper oleate, copper oxychloride, copper(II) sulfate, copper sulfate, basic, copper zinc chromate, cresol, cufraneb, cuprobam, cuprous oxide, cyazofamid, cyclafuramid, cyclic dithiocarbamate fungicides, cycloheximide, cyflufenamid, cymoxanil, cypendazole, cyproconazole, cyprodinil, dazomet, DBCP, debacarb, decafentin, dehydroacetic acid, dicarboximide fungicides, dichlofluanid, dichlone, dichlorophen, dichlorophenyl, dicarboximide fungicides, dichlozoline, diclobutrazol, diclocymet, diclomezine, dicloran, diethofencarb, diethyl pyrocarbonate, difenoconazole, diflumetorim, dimethirimol, dimethomorph, dimoxystrobin, diniconazole, dinitrophenol fungicides, dinobuton, dinocap, dinocton, dinopenton, dinosulfon, dinoterbon, diphenylamine, dipyrithione, disulfiram, ditalimfos, dithianon, dithiocarbamate fungicides, DNOC, dodemorph, dodicin, dodine, DONATODINE, drazoxolon, edifenphos, epoxiconazole, etaconazole,etem, ethaboxam, ethirimol, ethoxyquin, ethylmercury 2,3-dihydroxypropyl mercaptide, ethylmercury acetate, ethylmercury bromide, ethylmercury chloride, ethylmercury phosphate, etridiazole, famoxadone, fenamidone, fenaminosulf, fenapanil, fenarimol, fenbuconazole, fenfuram, fenhexamid, fenitropan, fenoxanil, fenpiclonil, fenpropidin, fenpropimorph, fentin, ferbam, ferimzone, fluazinam, fludioxonil, flumetover, flumorph, fluopicolide, fluoroimide, fluotrimazole, fluoxastrobin, fluquinconazole, flusilazole, flusulfamide, flutolanil, flutriafol, folpet, formaldehyde, fosetyl, fuberidazole, furalaxyl, furametpyr, furamide fungicides, furanilide fungicides, furcarbanil, furconazole, furconazole-cis, furfural, furmecyclox, furophanate, glyodin, griseofulvin, guazatine, halacrinate, hexachlorobenzene, hexachlorobutadiene, hexachlorophene, hexaconazole, hexylthiofos, hydrargaphen, hymexazol, imazalil, imibenconazole, imidazole fungicides, iminoctadine, inorganic fungicides, inorganic mercury fungicides, iodomethane, ipconazole, iprobenfos, iprodione, iprovalicarb, isoprothiolane, isovaledione, kasugamycin, kresoxim-methyl, lime sulphur, mancopper, mancozeb, maneb, mebenil, mecarbinzid, mepanipyrim, mepronil, mercuric chloride, mercuric oxide, mercurous chloride, mercury fungicides, metalaxyl, metalaxyl-M, metam, metazoxolon, metconazole, methasulfocarb, methfuroxam, methyl bromide, methyl isothiocyanate, methylmercury benzoate, methylmercury dicyandiamide, methylmercury pentachlorophenoxide, metiram, metominostrobin, metrafenone, metsulfovax, milneb, morpholine fungicides, myclobutanil, myclozolin, N-(ethylmercury)-p-toluenesulphonanilide, nabam, natamycin, nitrostyrene, nitrothal-isopropyl, nuarimol, OCH, octhilinone, ofurace, organomercury fungicides, organophosphorus fungicides, organotin fungicides, orysastrobin, oxadixyl, oxathiin fungicides, oxazole fungicides, oxine copper, oxpoconazole, oxycarboxin, pefurazoate, penconazole, pencycuron, pentachlorophenol, penthiopyrad, phenylmercuriurea, phenylmercury acetate, phenylmercury chloride, phenylmercury derivative of pyrocatechol, phenylmercury nitrate, phenylmercury salicylate, phenylsulfamide fungicides, phosdiphen, phthalide, phthalimide fungicides, picoxystrobin, piperalin, polycarbamate, polymeric dithiocarbamate fungicides, polyoxins, polyoxorim, polysulfide fungicides, potassium azide, potassium polysulfide, potassium thiocyanate, probenazole, prochloraz, procymidone, propamocarb, propiconazole, propineb, proquinazid, prothiocarb, prothioconazole, pyracarbolid, pyraclostrobin, pyrazole fungicides, pyrazophos, pyridine fungicides, pyridinitril, pyrifenox, pyrimethanil, pyrimidine fungicides, pyroquilon, pyroxychlor, pyroxyfur, pyrrole fungicides, quinacetol, quinazamid, quinconazole, quinoline fungicides, quinone fungicides, quinoxaline fungicides, quinoxyfen, quintozene, rabenzazole, salicylanilide, silthiofam, simeconazole, sodium azide, sodium orthophenylphenoxide, sodium pentachlorophenoxide, sodium polysulfide, spiroxamine, streptomycin, strobilurin fungicides, sulfonanilide fungicides, sulfur, sultropen, TCMTB, tebuconazole, tecloftalam, tecnazene, tecoram, tetraconazole, thiabendazole, thiadifluor, thiazole fungicides, thicyofen, thifluzamide, thiocarbamate fungicides, thiochlorfenphim, thiomersal, thiophanate, thiophanate-methyl, thiophene fungicides, thioquinox, thiram, tiadinil, tioxymid, tivedo, tolclofosmethyl, tolnaftate, tolylfluanid, tolylmercury acetate, triadimefon, triadimenol, triamiphos, triarimol, triazbutil, triazine fungicides, triazole fungicides, triazoxide, tributyltin oxide, trichlamide, tricyclazole, tridemorph, trifloxystrobin, triflumizole, triforine, triticonazole, unclassified fungicides, undecylenic acid, uniconazole, urea fungicides, validamycin, valinamide fungicides, vinclozolin, zarilamid, zinc naphthenate, zineb, ziram, zoxamide and their mixtures.
Herbicides: An herbicide is a pesticide used to kill unwanted plants. Selective herbicides kill specific targets while leaving the desired crop relatively unharmed. Some of these act by interfering with the growth of the weed and are often based on plant hormones. Herbicides used to clear waste ground are nonselective and kill all plant material with which they come into contact. Herbicides are widely used in agriculture and in landscape turf management. They are applied in total vegetation control (TVC) programs for maintenance of highways and railroads. Smaller quantities are used in forestry, pasture systems, and management of areas set aside as wildlife habitat. In the following, a number of suitable herbicides are compiled:
   ∘ 2,4-D, a broadleaf herbicide in the phenoxy group used in turf and in no-till field crop production. Now mainly used in a blend with other herbicides that act as synergists, it is the most widely used herbicide in the world, third most commonly used in the United States. It is an example of synthetic auxin (plant hormone).
   ∘ Atrazine, a triazine herbicide used in corn and sorghum for control of broadleaf weeds and grasses. It is still used because of its low cost and because it works as a synergist when used with other herbicides, it is a photosystem II inhibitor.
   ∘ Clopyralid, a broadleaf herbicide in the pyridine group, used mainly in turf, rangeland, and for control of noxious thistles. Notorious for its ability to persist in compost. It is another example of synthetic auxin.
   ∘ Dicamba, a persistent broadleaf herbicide active in the soil, used on turf and field corn. It is another example of synthetic auxin.
   ∘ Glyphosate, a systemic nonselective (it kills any type of plant) herbicide used in no-till burn down and for weed control in crops that are genetically modified to resist its effects. It is an example of a EPSPs inhibitor.
   ∘ Imazapyr, a non-selective herbicide used for the control of a broad range of weeds including terrestrial annual and perennial grasses and broadleaved herbs, woody species, and riparian and emergent aquatic species.
   ∘ Imazapic, a selective herbicide for both the pre- and post-emergent control of some annual and perennial grasses and some broadleaf weeds. Imazapic kills plants by inhibiting the production of branched chain amino acids (valine, leucine, and isoleucine), which are necessary for protein synthesis and cell growth.
   ∘ Metoalachlor, a pre-emergent herbicide widely used for control of annual grasses in corn and sorghum; it has largely replaced atrazine for these uses.
   ∘ Paraquat, a nonselective contact herbicide used for no-till burn down and in aerial destruction of marijuana and coca plantings. More acutely toxic to people than any other herbicide in widespread commercial use.
   ∘ Picloram, a pyridine herbicide mainly used to control unwanted trees in pastures and edges of fields. It is another synthetic auxin.
   ∘ Triclopyr.
Insecticides: An insecticide is a pesticide used against insects in all developmental forms. They include ovicides and larvicides used against the eggs and larvae of insects. Insecticides are used in agriculture, medicine, industry and the household. In the following, suitable insecticides are mentioned:
   ∘ Chlorinated insecticides such as, for example, Camphechlor, DDT, Hexachloro¬cyclohexane, gamma-Hexachlorocyclohexane, Methoxychlor, Pentachlorophenol, TDE, Aldrin, Chlordane, Chlordecone, Dieldrin, Endosulfan, Endrin, Heptachlor, Mirex and their mixtures;
   ∘ Organophosphorus compounds such as, for example, Acephate, Azinphos-methyl, Ben¬sulide, Chlorethoxyfos, Chlorpyrifos, Chlorpyriphos-methyl, Diazinon, Dichlorvos (DDVP), Dicrotophos, Dimethoate, Disulfoton, Ethoprop, Fenamiphos, Fenitrothion, Fenthion, Fosthiazate, Malathion, Methamidophos, Methidathion, Methyl-parathion, Mevinphos, Naled, Omethoate, Oxydemeton-methyl, Parathion, Phorate, Phosalone, Phosmet, Phostebupirim, Pirimiphos-methyl, Profenofos, Terbufos, Tetrachlorvinphos, Tribufos, Trichlorfon and their mixture;
   ∘ Carbamates such as, for example, Aldicarb, Carbofuran, Carbaryl, Methomyl, 2-(1-Methylpropyl)phenyl methylcarbamate and their mixtures;
   ∘ Pyrethroids such as, for example, Allethrin, Bifenthrin, Deltamethrin, Permethrin, Resmethrin, Sumithrin, Tetramethrin, Tralomethrin, Transfluthrin and their mixtures;
   ∘ Plant toxin derived compounds such as, for example, Derris (rotenone), Pyrethrum, Neem (Azadirachtin), Nicotine, Caffeine and their mixtures.
Rodenticides: Rodenticides are a category of pest control chemicals intended to kill rodents. In the following, examples for suitable rodenticides are given:
   ∘ Anticoagulants, e.g. difenacoum, brodifacoum, flocoumafen, bromadiolone, difethialone, warfarin, coumatetralyl, chlorophacinone, diphacinone, coumachlor, coumafuryl and pindone;
   ∘ Metal phosphides;
   ∘ Phosphides; or
   ∘ Hypercalcemia, e.g. Calciferols (vitamins D), cholecalciferol (vitamin D3) and ergocalciferol (vitamin D2).
Miticides, moluscicides and nematicides: Miticides are pesticides that kill mites. Antibiotic miticides, carbamate miticides, formamidine miticides, mite growth regulators, organochlorine, permethrin and organophosphate miticides all belong to this category. Molluscicides are pesticides used to control mollusks, such as moths, slugs and snails. These substances include metaldehyde, methiocarb and aluminium sulfate. A nematicide is a type of chemical pesticide used to kill parasitic nematodes (a phylum of worm). A nematicide is obtained from a neem tree's seed cake; which is the residue of neem seeds after oil extraction. The neem tree is known by several names in the world but was first cultivated in India since ancient times.

The pesticide usually has a water-solubility up to 10 g/l, preferably up to 5 g/l, and more preferably up to 1 g/l.

The pesticide may be solid or liquid at 20 °C.

The pesticide usually does not contain nucleophilic groups selected from hydroxyl, thiol, primary nitrogen, secondary nitrogen and carbanion.

Preferred pesticides are herbicides, insecticides and fungicides, wherein herbicides are more preferred.

Examples of suitable herbicides are tepraloxydim, flufenacet, napropamid, isoxaben, fluazifop-P-butyl, metamitron, propyzamide, phenmedipham, clethodim, chloridazon, dimethenamid-P, and pendimethalin. A preferred example is dimethenamid-P.

Besides the before mentioned compounds the microcapsules of the present invention may also contain any desired blends of oils, as well as blends of oil and water in emulsified form. Any kind of emulsion (water-in-oil or oil-in-water, or multiple emulsions) is possible.

For this purpose emulsifiers are needed: The microcapsules according to the present invention might also contain one or more emulsifier. Suitable emulsifiers are, for example, nonionic surfactants from at least one of the following groups: products of the addition of 2 to 30 mol ethylene oxide and/or 0 to 5 mol propylene oxide onto linear C.sub.6-22 fatty alcohols, onto C.sub.12-22 fatty acids, onto alkyl phenols containing 8 to 15 carbon atoms in the alkyl group and onto alkylamines containing 8 to 22 carbon atoms in the alkyl group; alkyl oligoglycosides containing 8 to 22 carbon atoms in the alkyl group and ethoxylated analogs thereof; addition products of 1 to 15 mol ethylene oxide onto castor oil and/or hydrogenated castor oil; addition products of 15 to 60 mol ethylene oxide onto castor oil and/or hydrogenated castor oil; partial esters of glycerol and/or sorbitan with unsaturated, linear or saturated, branched fatty acids containing 12 to 22 carbon atoms and/or hydroxycarboxylic acids containing 3 to 18 carbon atoms and addition products thereof onto 1 to 30 mol ethylene oxide; partial esters of polyglycerol (average degree of self-condensation 2 to 8), polyethylene glycol (molecular weight 400 to 5,000), trimethylolpropane, pentaerythritol, sugar alcohols (for example sorbitol), alkyl glucosides (for example methyl glucoside, butyl glucoside, lauryl glucoside) and polyglucosides (for example cellulose) with saturated and/or unsaturated, linear or branched fatty acids containing 12 to 22 carbon atoms and/or hydroxycarboxylic acids containing 3 to 18 carbon atoms and addition products thereof onto 1 to 30 mol ethylene oxide; mixed esters of pentaerythritol, fatty acids, citric acid and fatty alcohol and/or mixed esters of fatty acids containing 6 to 22 carbon atoms, methyl glucose and polyols, preferably glycerol or polyglycerol, mono-, di- and trialkyl phosphates and mono-, di- and/or tri-PEG-alkyl phosphates and salts thereof, wool wax alcohols, polysiloxane/polyalkyl/polyether copolymers and corresponding derivatives, block copolymers, for example Polyethyleneglycol-30 Dipolyhydroxystearate; polymer emulsifiers, for example Pemulen types (TR-1, TR-2) of Goodrich; polyalkylene glycols and glycerol carbonate and ethylene oxide addition products.

It is likewise possible for the ingredients to migrate from the core of the microcapsules (i.e. the oil and/or further materials present in the core) into the shell.

The invention further provides aqueous dispersions comprising 5 to 50% by weight, based on the total weight of the dispersion, preferably from 15 to 40% by weight, of microcapsules which can be produced by the above process. A further preferred range is between 20 and 35% by weight. These aqueous dispersions are preferably obtainable directly from the process described above.

The microcapsule dispersions which are obtainable by the present process can be used in a large number of different applications, depending on the type of oil.

The microcapsules may be present in form of an **agrochemical composition**. An agrochemical composition comprises a pesticidally effective amount of the microcapsules. The term "effective amount" denotes an amount of the composition or of the microcapsules, which is sufficient for combating undesired plant growth, and/or infestation of plants by insects and/or infestation of plants by phytopathogenic and which does not result in a substantial damage to the treated plants. Such an amount can vary in a broad range and is dependent on various factors, such as the fungal species to be controlled, the treated cultivated plant or material, the climatic conditions and the specific pesticide used.

The microcapsules of the invention can be formulated in a variety of agrochemical compositions. Examples for agrochemical composition types microcapsules formulations (e.g. CS, ZC), pastes, pastilles, wettable powders or dusts (e.g. WP, SP, WS, DP, DS), pressings (e.g. BR, TB, DT), granules (e.g. WG, SG, GR, FG, GG, MG), insecticidal articles (e.g. LN), as well as gel formulations for the treatment of plant propagation materials such as seeds (e.g. GF). These and further compositions types are defined in the " Catalogue of pesticide formulation types and international coding system" , Technical Monograph No. 2, 6th Ed. May 2008, CropLife International.

The compositions are **prepared** in a known manner, such as described by Mollet and Grubemann, Formulation technology, Wiley VCH, Weinheim, 2001; or Knowles, New developments in crop protection product formulation, Agrow Reports DS243, T&F Informa, London, 2005.

The agrochemical compositions may contain the microcapsules and auxiliaries.

Suitable **auxiliaries** are solvents, liquid carriers, solid carriers or fillers, surfactants, dispersants, emulsifiers, wetters, adjuvants, solubilizers, penetration enhancers, protective colloids, adhesion agents, thickeners, humectants, repellents, attractants, feeding stimulants, compatibilizers, bactericides, anti-freezing agents, anti-foaming agents, colorants, tackifiers and binders.

Suitable **solvents** and liquid carriers are usually water.

Suitable **surfactants** are surface-active compounds, such as anionic, cationic, nonionic and amphoteric surfactants, block polymers, polyelectrolytes, and mixtures thereof. Such surfactants can be used as emusifier, dispersant, solubilizer, wetter, penetration enhancer, protective colloid, or adjuvant. Examples of surfactants are listed in McCutcheon' s, Vol.1: Emulsifiers & Detergents, McCutcheon' s Directories, Glen Rock, USA, 2008 (International Ed. or North American Ed.).

Suitable **anionic** surfactants are alkali, alkaline earth or ammonium salts of sulfonates, sulfates, phosphates, carboxylates, and mixtures thereof. Examples of sulfonates are alkylarylsulfonates, diphenylsulfonates, alpha-olefin sulfonates, lignine sulfonates, sulfonates of fatty acids and oils, sulfonates of ethoxylated alkylphenols, sulfonates of alkoxylated arylphenols, sulfonates of condensed naphthalenes, sulfonates of dodecyl- and tridecylbenzenes, sulfonates of naphthalenes and alkylnaphthalenes, sulfosuccinates or sulfosuccinamates. Examples of sulfates are sulfates of fatty acids and oils, of ethoxylated alkylphenols, of alcohols, of ethoxylated alcohols, or of fatty acid esters. Examples of phosphates are phosphate esters. Examples of carboxylates are alkyl carboxylates, and carboxylated alcohol or alkylphenol ethoxylates.

Suitable **nonionic** surfactants are alkoxylates, N-subsituted fatty acid amides, amine oxides, esters, sugar-based surfactants, polymeric surfactants, and mixtures thereof. Examples of alkoxylates are compounds such as alcohols, alkylphenols, amines, amides, arylphenols, fatty acids or fatty acid esters which have been alkoxylated with 1 to 50 equivalents. Ethylene oxide and/or propylene oxide may be employed for the alkoxylation, preferably ethylene oxide. Examples of N-subsititued fatty acid amides are fatty acid glucamides or fatty acid alkanolamides. Examples of esters are fatty acid esters, glycerol esters or monoglycerides. Examples of sugar-based surfactants are sorbitans, ethoxylated sorbitans, sucrose and glucose esters or alkylpolyglucosides. Examples of polymeric surfactants are home- or copolymers of vinylpyrrolidone, vinylalcohols, or vinylacetate.

Suitable **cationic** surfactants are quaternary surfactants, for example quaternary ammonium compounds with one or two hydrophobic groups, or salts of long-chain primary amines. Suitable **amphoteric** surfactants are alkylbetains and imidazolines. Suitable **block polymers** are block polymers of the A-B or A-B-A type comprising blocks of polyethylene oxide and polypropylene oxide, or of the A-B-C type comprising alkanol, polyethylene oxide and polypropylene oxide. Suitable **polyelectrolytes** are polyacids or polybases. Examples of polyacids are alkali salts of polyacrylic acid or polyacid comb polymers. Examples of polybases are polyvinylamines or polyethyleneamines.

Suitable **adjuvants** are compounds, which have a neglectable or even no pesticidal activity themselves, and which improve the biological performance of the pesticide on the target. Examples are surfactants, mineral or vegetable oils, and other auxilaries. Further examples are listed by Knowles, Adjuvants and additives, Agrow Reports DS256, T&F Informa UK, 2006, chapter 5.

Suitable **thickeners** are polysaccharides (e.g. xanthan gum, carboxymethylcellulose), anorganic clays (organically modified or unmodified), polycarboxylates, and silicates.

Suitable **bactericides** are bronopol and isothiazolinone derivatives such as alkylisothiazolinones and benzisothiazolinones.

Suitable **anti-freezing agents** are ethylene glycol, propylene glycol, urea and glycerin.

Suitable **anti-foaming agents** are silicones, long chain alcohols, and salts of fatty acids.

Suitable **colorants** (e.g. in red, blue, or green) are pigments of low water solubility and watersoluble dyes. Examples are inorganic colorants (e.g. iron oxide, titan oxide, iron hexacyanoferrate) and organic colorants (e.g. alizarin-, azo- and phthalocyanine colorants). Suitable **tackifiers or binders** are polyvinylpyrrolidons, polyvinylacetates, polyvinyl alcohols, polyacrylates, biological or synthetic waxes, and cellulose ethers.

The agrochemical compositions generally comprise between 0.01 and 95%, preferably between 0.1 and 90%, and in particular between 0.5 and 75%, by weight of the pesticide.

The agrochemical composition may be employed for the purposes of **treatment of plant propagation materials**, particularly seeds. The compositions in question give, after two-to-tenfold dilution, active substance concentrations of from 0.01 to 60% by weight, preferably from 0.1 to 40% by weight, in the ready-to-use preparations. Application can be carried out before or during sowing. Methods for applying the pesticide and compositions thereof, respectively, on to plant propagation material, especially seeds include dressing, coating, pelleting, dusting, soaking and in-furrow application methods of the propagation material. Preferably, the agrochemical compositions, respectively, is applied on to the plant propagation material by a method such that germination is not induced, e. g. by seed dressing, pelleting, coating and dusting.

When employed in plant protection, the **amounts of pesticide applied** are, depending on the kind of effect desired, from 0.001 to 2 kg per ha, preferably from 0.005 to 2 kg per ha, more preferably from 0.05 to 0.9 kg per ha, and in particular from 0.1 to 0.75 kg per ha.
In treatment of plant propagation materials such as seeds, e. g. by dusting, coating or drenching seed, amounts of active substance of from 0.1 to 1000 g, preferably from 1 to 1000 g, more preferably from 1 to 100 g and most preferably from 5 to 100 g, per 100 kilogram of plant propagation material (preferably seeds) are generally required.
When used in the protection of materials or stored products, the amount of active substance applied depends on the kind of application area and on the desired effect. Amounts customarily applied in the protection of materials are 0.001 g to 2 kg, preferably 0.005 g to 1 kg, of active substance per cubic meter of treated material.

Various types of oils, wetters, adjuvants, fertilizer, or micronutrients, and further pesticides (e.g. herbicides, insecticides, fungicides, growth regulators, safeners) may be added to the the agrochemical compositions comprising them as premix or, if appropriate not until immediately prior to use (**tank mix**). These agents can be admixed with the compositions according to the invention in a weight ratio of 1:100 to 100:1, preferably 1:10 to 10:1.

The **user applies** the agrochemical composition usually from a predosage device, a knapsack sprayer, a spray tank, a spray plane, or an irrigation system. Usually, the agrochemical composition is made up with water, buffer, and/or further auxiliaries to the desired application concentration and the ready-to-use spray liquor or the agrochemical composition according to the invention is thus obtained. Usually, 20 to 2000 liters, preferably 50 to 400 liters, of the ready-to-use spray liquor are applied per hectare of agricultural useful area.

The present inventnion further relates to a method of controlling phytopathogenic fungi and/or undesired plant growth and/or unde-sired insect or mite attack and/or for regulating the growth of plants, wherein the microcapsules are allowed to act on the respective pests, their environment or the crop plants to be protected from the respective pest, on the soil and/or on undesired plants and/or on the crop plants and/or on their environment.

Examples of suitable crop plants are cereals, for example wheat, rye, barley, triticale, oats or rice; beet, for example sugar or fodder beet; pome fruit, stone fruit and soft fruit, for example apples, pears, plums, peaches, almonds, cherries, strawberries, raspberries, currants or gooseberries; legumes, for example beans, lentils, peas, lucerne or soybeans; oil crops, for example oilseed rape, mustard, olives, sunflowers, coconut, cacao, castor beans, oil palm, peanuts or soybeans; cucurbits, for example pumpkins/squash, cucumbers or melons; fiber crops, for example cotton, flax, hemp or jute; citrus fruit, for example oranges, lemons, grapefruit or tangerines; vegetable plants, for example spinach, lettuce, asparagus, cabbages, carrots, onions, tomatoes, potatoes, pumpkin/squash or capsicums; plants of the laurel family, for example avocados, cinnamon or camphor; energy crops and industrial feedstock crops, for example maize, soybeans, wheat, oilseed rape, sugar cane or oil palm; maize; tobacco; nuts; coffee; tea; bananas; wine (dessert grapes and grapes for vinification); hops; grass, for example turf; sweetleaf (Stevia rebaudania); rubber plants and forest plants, for example flowers, shrubs, deciduous trees and coniferous trees, and propagation material, for example seeds, and harvested produce of these plants.

The term crop plants also includes those plants which have been modified by breeding, mutagenesis or recombinant methods, including the biotechnological agricultural products which are on the market or in the process of being developed. Genetically modified plants are plants whose genetic material has been modified in a manner which does not occur under natural conditions by hybridizing, mutations or natural recombination (i.e. recombination of the genetic material). Here, one or more genes will, as a rule, be integrated into the genetic material of the plant in order to improve the plant's properties. Such recombinant modifications also comprise posttranslational modifications of proteins, oligo- or polypeptides, for example by means of glycosylation or binding polymers such as, for example, prenylated, acetylated or farnesylated residues or PEG residues.

The present invention further relates to seed containing the microcapsules.

The present invention has various advantages: The invention increases the stability of the formulation within broad range of temperatures; the microcapsules may be loaded with both oil and pesticide, and optionally adjuvants; the microcapsules have a increased rainfastness; there is a reduced toxicological effect for the worker and users; the microcapsules are very stable against UV-light or sunlight; the microcapsules have a high physical stability; the microcapsules have a excellent biodelivery; the microcapsules have a very low toxicology (e.g. no eye irritation); the microcapsules have a low contact angle of the sprayed drops on leaves; the microcapsules have a high spreading on leaves. The invention allows for a very slow release of the pesiticide, e.g. over at least 3 weeks. The invention allows for a reduced phytotoxicity of pesiticide; it is possible to mix the microcapsules with water soluble or dispersed pesticides; or it is possible to mix the microcapsules comprising a pesticide with microcapsules comprising another pesticide; or it has a high efficacy.

The examples below give further illustration of the invention, which is not, however, restricted to these examples.

### Example 1 - Dimethenamid-P microcapsules

Premix (I) was prepared from 50 g of carboxyl group-modified anionic PVA (Kuraray Poval 25-88 KL from Kuraray with hydrolysis degree 85% - 90% and Brookfield viscosity 20.0-30.0 mPas at 4% in water at 20 °C) and 668 g of water.
Premix (II) was prepared from 611 g of dimethenamid-P (DMTA-P, S-2-chloro-N-(2,4-dimethyl-3-thienyl)-N-(2-methoxy-1-methylethyl)-acetamide, liquid, water-insoluble), 78 g of dicyclohexylmethane diisocyanate and 22 g of Bayhydur® XP 2547 (anionic water-dispersible polyisocyanate based on hexamethylene diisocyanate; NCO about 22.5%, equivalent weight average about 182, monomeric isocyanate <0.5%). These two premixes were combined and emulsified with the help of a stirrer for 30 minutes at room temperature at a speed of 700 rpm. Then, at room temperature a solution of 40 g of Polyethyleneimine A (average molelcular weight 2000, viscosity 10000-20000 mPas at 20 °C, pour point -9 °C, water content <2.0%) in 75 g of water was added.
The reaction mixture was then subjected to the following temperature program: heating to 60°C in 60 minutes, maintaining this temperature for 60 minutes, then 60 minutes at 70°C, 60 minutes at 80°C and finally 60 minutes at 85°C. The mixture was then cooled to room temperature, giving the desired microcapsule dispersion with a fraction of nonvolatile components of 39.2 % and a particle size d50= 5µm.

### Example 2 (Comparative) - Variation of capsule wall

Premix(I) was prepared from 12 g of sodium lignosulfonate (Reax® 88B) and 488 g of water. Premix (II) was prepared from 442 g of dimethenamid-P and 49 g of solvent free polyisocyanate based on 4,4'-diphenylmethane diisocyanate (MDI) (average functionality of 2,7, NCO content 32 g/100 g). These two premixes were combined and emulsified with the help of a high shear homogenizer for 2 minutes at room temperature at a speed of 7000 rpm. Afterwards, the emulsion was further stirred with a Mig stirrer at room temperature with a stirring speed of 700 rpm. 75 g of hexamethylenediamine was added over the course of 2 minutes. The reaction mixture was then stirred at room temperature for 2 hours to give the desired microcapsule dispersion with a fraction of nonvolatile components of 54.2% and a particle size d50= 7µm

The release rate of the pesticide from the microcapsules was tested stirring the capsules at room temperature in water containing 10 wt% surfactant to solubilize the released pesticide in water. The amount of released pesticide was determined by quantitative HPLC and compared to the release rate of the microcapsules of Example 1. It was demonstrated that the use of the shell wall according to Example 1 resulted in a slower release of the pesticide.

**Table 1: Released pesticide from microcapsules (% of total pesticide)**

| | 10 min | 5 hours | 1 day | 3 days | 7 days | 14 days | 21 days |
|---|---|---|---|---|---|---|---|
| Example 2 (Comparative) | 6,5 | 8,7 | 16,2 | 19,8 | 44,1 | 61,6 | - |
| Example 1 | 0 | 0,4 | 1,5 | 4,5 | 9,5 | 17,1 | 28,5 |

### Example 3 - Acetochlor microcapsules

The synthesis of Example 1 is repeated with acetochlor instead of dimethenamid-P at the same concentration. Microcapsules with a similar particle size are obtained.

### Example 4 - Metazachlor microcapsules

The synthesis of Example 1 is repeated with metazachlor instead of dimethenamid-P at the same concentration. Microcapsules with a similar particle size are obtained.

### Example 5 - Core with pesticide and oily solvent

The preparation was made as decribed in Example 1, except that premix (II) was prepared from 489 g of Dimethenamid-P, 122 g of aromatic hydrocarbon solvent (distillation range 232 to 277 °C, aromatic content >99 vol%, viscosity 2.74 mm²/s at 25 °C), 78 g of dicyclohexylmethane diisocyanate and 22 g of Bayhydur® XP 2547. The resulting microcapsule dispersion had a fraction of nonvolatile components of 39.8 % and a particle size d50= 4µm.

### Example 6 (Comparative) - Neutral protective colloid

The preparation was made as decribed in Example 5, except that neutral polyvinylpyrrolidone (PVP K90) was used instead of carboxyl group-modified anionic PVA at the same concentration.

The release rate of the pesticide from the microcapsules of Example 6 was tested as in Example 2 and compared to the release rate of the microcapsules of Example 5. It was demonstrated that the use of the anionic protective colloid resulted in a slower release of the pesticide.

**Table 2: Released pesticide from microcapsules (% of total pesticide)**

| | 10 min | 5 hours | 1 day | 3 days | 7 days | 14 days |
|---|---|---|---|---|---|---|
| Example 6 | 0,1 | 0,9 | 4,2 | 7,8 | 22,4 | 40,0 |
| (neutral protective colloid) | | | | | | |
| Example 5 | 0,1 | 0,4 | 1,3 | 3,1 | 6,6 | 12,4 |
| (anionic protective colloid) | | | | | | |

### Example 7 (Comparative) - Variation of capsule wall

Premix(I) was prepared from 12 g of sodium lignosulfonate (Reax® 88B) and 480 g of water. Premix (II) was prepared from 370 g of dimethenamid-P, 96 g aromatic hydrocarbon solvent (distillation range 232 to 277 °C, aromatic content >99 vol%, viscosity 2.74 mm²/s at 25 °C), and 23 g of solvent free polyisocyanate based on 4,4'-diphenylmethane diisocyanate (MDI) (average functionality of 2,7, NCO content 32 g/100 g). These two premixes were combined and emulsified with the help of a high shear homogenizer for 2 minutes at room temperature at a speed of 7000 rpm. Afterwards, the emulsion was further stirred at room temperature with a stirring speed of 700 rpm. 9 g of hexamethylenediamine was added over the course of 2 minutes. The reaction mixture was then stirred at room temperature for 2 hours to give the desired microcapsule dispersion with a fraction of nonvolatile components of 51% and a particle size d50= 8µm

The release rate of the pesticide from the microcapsules of Example 7 was tested (cf. Example 2) and compared to the release rate of the microcapsules of Example 5. It was demonstrated that the use of the shell wall according to Example 5 resulted in a slower release of the pesticide.

**Table 3: Released pesticide from microcapsules (% of total pesticide)**

| | 10 min | 5 hours | 1 day | 3 days | 7 days | 14 days |
|---|---|---|---|---|---|---|
| Example 7 (Comparative) | 2,8 | 4,9 | 7,7 | 16,9 | 28,7 | 43,7 |
| Example 5 | 0,1 | 0,4 | 1,3 | 3,1 | 6,6 | 12,4 |

### Example 8 - Cinmethylin microcapsules

The preparation was made as decribed in Example 1, except that premix (II) was prepared from 611 g of Cinmethylin (liquid, boiling point >300 °C, solubility in water about 0.06 g/l at 20 °C), 78 g of dicyclohexylmethane diisocyanate and 22 g of Bayhydur® XP 2547. The resulting microcapsule dispersion had a fraction of nonvolatile components of 39.5 % and a particle size d50= 12 µm.

### Example 9 - Sufonic acid modified protective colloid

The preparation was made as decribed in Example 1, except that premix (I) was prepared from 50 g of sulfonic acid group modified anionic PVA (Gohseran L-3266, Nippon Gohsei, with hydrolysis degree 86.5% - 89.5mol% and viscosity of 2.3-2.7 mPas at 20 °C, 4% in water) and 668 g of water. The resulting microcapsule dispersion had a fraction of nonvolatile components of 40.0 % and a particle size d50= 10 µm.

### Example 10 - Phytotoxicity

In greenhouse trials the postemergence crop safety and phytotoxicity of dimethenamid-P on soybean and cotton. The plants were treated with the aqueous tank mix via an AIXR Teejet spray nozzle at the V2 (soybean) or 2 (cotton) growth stage at an application rate of 15 gallons per acre (947 g/ha dimethenamid-P). In all treatments glyphosate was included at a rate of 1120 g ae/ha. The damage on the plants was visually assessed 3 and 14 days after treatment (DAT). The results in Tables 4 and 5 demonstrated that the inventive Example 1 caused the least injury on the crop plants compared to comparative Examples 2 and 7. During the tests there was no nozzle clothing observed.

**Table 4: Phytotoxicity (%) of dimethenamid-P on soybean**

| | 3 DAT | 14 DAT |
|---|---|---|
| Example 1 | 11 | 21 |
| Example 2 (comparative) | 18 | 28 |
| Example 7 (comparative) | 17 | 30 |

**Table 5: Phytotoxicity (%) of dimethenamid-P on cotton**

| | 3 DAT | 14 DAT |
|---|---|---|
| Example 1 | 8 | 11 |
| Example 2 (comparative) | 24 | 27 |
| Example 7 (comparative) | 28 | 32 |

### Example 11 - Phytotoxicity and efficacy

In field trials in Brazil the phytotoxicity of dimethenamid-P on soybean and its efficacy on weeds was tested. The plants were treated with the aqueous tank mix at the V2 growth stage at an application rate of 200 l/ha (840 g/ha dimethenamid-P). In all treatments glyphosate was included at a rate of 1080 g/ha or 2160 g/ha, respectively. The damage on the plants was visually assessed 7 days after treatment (DAT). For control some plants were not treated. During the tests there was no nozzle clothing observed.

The results in Tables 6 and 7 demonstrated that the inventive Example 1 caused the least injury on the crop plants compared to comparative Examples 2 and 7. The results in Tables 6 and 7 further demonstrated that the inventive Example 1 has similar or even higher efficacy against weeds compared to comparative Examples 2 and 7.

The efficacy on the following weeds was tested:

| | |
|---|---|
| BRADC: | Brachiaria decumbens, Surinam grass |
| ELEIN: | Eleusine indica, Goosegras |
| COMBE: | Commelina benghalensis, Bengal day flower |
| EPHHL: | Euphorbia heterophylla, painted spurge |
| IPOTR: | Ipomoea triloba, three-lobe morning glory |
| DIGHO: | Digitaria horizontalis, tiende capote |

**Table 6: Phytotoxicity and efficacy (incl. 1080 g/ha glyphosate)**

| | Untreated Control | Example 1 | Example 2 (comparative) | Example 7 (comparative) |
|---|---|---|---|---|
| Phytotoxicity | 13,0 | 7,7 | 11,7 | 11,3 |
| BRADC | 4,7 | 91,7 | 96,0 | 91,7 |
| ELEIN | 1,7 | 100 | 100 | 100 |
| COMBE | 1,0 | 91,7 | 86,7 | 93,3 |
| EPHHL | 5,3 | 61,7 | 60,0 | 48,3 |
| IPOTR | 3,3 | 58,3 | 53,3 | 55,0 |
| DIGHO | 1,0 | 100 | 100 | 100 |

**Table 7: Phytotoxicity and efficacy (incl. 2160 g/ha glyphosate)**

| | Untreated Control | Example 1 | Example 2 (comparative) | Example 7 (comparative) |
|---|---|---|---|---|
| Phytotoxicity | 13,0 | 7,7 | 11,7 | 11,3 |
| BRADC | 4,7 | 93,3 | 90,0 | 89,3 |
| ELEIN | 1,7 | 100 | 100 | 100 |
| COMBE | 1,0 | 92,7 | 90 | 88,3 |
| EPHHL | 5,3 | 66,7 | 70 | 63,3 |
| IPOTR | 3,3 | 60,0 | 60 | 65,0 |
| DIGHO | 1,0 | 100 | 98,3 | 100 |

## Claims

1. A process for producing microcapsules which contain a shell and a core of a liquid water-insoluble material, where
(a) a premix (I) is prepared from water and a protective colloid;
(b) a further premix (II) is prepared from the water-insoluble material and at least bifunctional isocyanate (A) or a mixture of two or more different isocyanates containing (A);
(c) the two premixes (I) and (II) are mixed together until an emulsion is formed;
(d) at least a bifunctional amine is then poured into the emulsion from step (c); and
(e) the emulsion is then heated until the microcapsules are formed; and
where the liquid water-insoluble material comprises a pesticide,
where the protective colloid is a polyvinyl alcohol copolymer having hydrolysis degrees from 60 to 99.9 %,
where the polyvinyl alcohol copolymer contains comonomers with anionic groups, and wherein the isocyanate (A) is selected from alicyclic or aliphatic isocyanates.

2. The process as claimed in claim 1, wherein the polyvinyl alcohol copolymer contains comonomers with anionic groups selected from carboxyl- and/or sulfonic acid groups.

3. The process as claimed in claim 1 or 2, wherein the polyvinyl alcohol copolymer contains 0.1 to 30 mol% of the comonomers with anionic groups.

4. The process as claimed in at least one of claims 1 to 3, wherein the polyvinyl alcohol copolymer is used with amounts from 0.1 to 20% by weight, based on the weight of the microcapsules.

5. The process as claimed in at feast of the claims 1 to 4, wherein the water-insoluble material comprises a pesticide blended with an oily solvent.

6. The process as claimed in at least one of claims 1 to 5, wherein the isocyanate (A) is selected from hexamethylene diisocyanate or dicyclohexylmethane diisocyanates.

7. The process as claimed in at least one of claims 1 to 6, wherein a mixture of isocyanate (A) and an anionically modified isocyanate (B) is used, wherein the anionically modified diisocyanates (B) are selected from the group which contain at least one sulfonic acid group in the molecule.

8. The process as claimed in claim 7, wherein the weight ratio between the isocyanates (A) and (B) is in the range from 10:1 to 1:10.

9. The process as claimed in at least one of claims 1 to 8, wherein the at least bifunctional amine used is a polyethyleneimine.

10. The process as claimed in at least one of claims 1 to 9, where the pesticide has a water-solubility up to 10 g/l at 20 °C.

11. The process as claimed in at least one of claims 1 to 10, wherein the core-shell ratio (w/w) of the microcapsules is 20:1 to 1:10.

12. The process as claimed in at least one of claims 1 to 11, wherein microcapsules have a diameter from 1 to 30 µm.

13. The process as claimed in at least one of claims 1 to 11, wherein the emulsion is then heated to at least 50 °C.

14. A method of controlling phytopathogenic fungi and/or undesired plant growth and/or undesired insect or mite attack and/or for regulating the growth of plants, wherein the microcapsules obtainable by a process as defined in any of claims 1 to 13 are allowed to act on the respective pests, their environment or the crop plants to be protected from the respective pest, on the soil and/or on undesired plants and/or on the crop plants and/or on their environment.

## Patentansprüche

1. Verfahren zur Herstellung von eine Hülle und einen Kern eines flüssigen wasserunlöslichen Materials enthaltenden Mikrokapseln, bei dem man
(a) aus Wasser und einem Schutzkolloid eine Vormischung (I) herstellt,
(b) aus dem wasserunlöslichen Material und mindestens bifunktionellem Isocyanat (A) oder einer Mischung von zwei oder mehr verschiedenen (A) enthaltenden Isocyanaten eine weitere Vormischung (II) herstellt,
(c) die beiden Vormischungen (I) und (II) bis zur Bildung einer Emulsion mischt,
(d) dann mindestens ein bifunktionelles Amin in die Emulsion aus Schritt (c) gießt und
(e) anschließend die Emulsion bis zur Bildung der Mikrokapseln erhitzt, und
wobei das flüssige wasserunlösliche Material ein Pestizid umfasst,
wobei es sich bei dem Schutzkolloid um ein Polyvinylalkoholcopolymer mit einem Hydrolysegrad von 60 bis 99,9% handelt,
wobei das Polyvinylalkoholcopolymer Comonomere mit anionischen Gruppen enthält und
wobei das Isocyanat (A) aus alicyclischen oder aliphatischen Isocyanaten ausgewählt ist.

2. Verfahren nach Anspruch 1, wobei das Polyvinylalkoholcopolymer Comonomere mit aus Carboxyl- und/oder Sulfonsäuregruppen ausgewählten anionischen Gruppen enthält.

3. Verfahren nach Anspruch 1 oder 2, wobei das Polyvinylalkoholcopolymer 0,1 bis 30 mol-% der Comonomere mit anionischen Gruppen enthält.

4. Verfahren nach mindestens einem der Ansprüche 1 bis 3, wobei das Polyvinylalkoholcopolymer in Mengen von 0,1 bis 20 Gew.-%, bezogen auf das Gewicht der Mikrokapseln, eingesetzt wird.

5. Verfahren nach mindestens einem der Ansprüche 1 bis 4, wobei das wasserunlösliche Material ein mit einem öligen Lösungsmittel gemischtes Pestizid umfasst.

6. Verfahren nach mindestens einem der Ansprüche 1 bis 5, wobei das Isocyanat (A) aus Hexamethylendiisocyanat oder Dicyclohexylmethandiisocyanaten ausgewählt ist.

7. Verfahren nach mindestens einem der Ansprüche 1 bis 6, bei dem man eine Mischung von Isocyanat (A) und einem anionisch modifizierten Isocyanat (B) einsetzt, wobei die anionisch modifizierten Diisocyanate (B) aus der mindestens eine Sulfonsäuregruppe im Molekül enthaltenden Gruppe ausgewählt sind.

8. Verfahren nach Anspruch 7, wobei das Gewichtsverhältnis der Isocyanate (A) zu den Isocyanaten (B) im Bereich von 10:1 bis 1:10 liegt.

9. Verfahren nach mindestens einem der Ansprüche 1 bis 8, wobei es sich bei dem eingesetzten mindestens bifunktionellen Amin um ein Polyethylenimin handelt.

10. Verfahren nach mindestens einem der Ansprüche 1 bis 9, wobei das Pestizid bei 20°C eine Löslichkeit im Wasser von bis zu 10 g/l aufweist.

11. Verfahren nach mindestens einem der Ansprüche 1 bis 10, wobei das Kern/Hülle-Verhältnis (w/w) der Mikrokapseln 20:1 bis 1:10 beträgt.

12. Verfahren nach mindestens einem der Ansprüche 1 bis 11, wobei die Mikrokapseln einen Durchmesser von 1 bis 30 µm aufweisen.

13. Verfahren nach mindestens einem der Ansprüche 1 bis 11, wobei die Emulsion dann auf mindestens 50°C erhitzt wird.

14. Verfahren zur Bekämpfung phytopathogener Pilze und/oder unerwünschten Pflanzenwuchses und/oder unerwünschten Insekten- oder Milbenbefalls und/oder zur Steuerung des Wachstums von Pflanzen, bei dem man die nach dem wie in einem der Ansprüche 1 bis 13 definierten Verfahren erhältlichen Mikrokapseln auf die jeweiligen Schädlinge, ihre Umgebung oder die gegen die jeweiligen Schädlinge zu schützenden Kulturpflanzen, auf den Boden und/oder auf unerwünschte Pflanzen und/oder auf die Kulturpflanzen und/oder auf ihre Umgebung eingewirken lässt.

## Revendications

1. Procédé de production de microcapsules qui contiennent une enveloppe et un noyau d'un matériau liquide insoluble dans l'eau, où
(a) un prémélange (I) est préparé à partir d'eau et d'un colloïde protecteur ;
(b) un prémélange supplémentaire (II) est préparé à partir du matériau insoluble dans l'eau et d'un isocyanate au moins bifonctionnel (A) ou un mélange de deux ou plus de deux isocyanates différents contenant (A) ;
(c) les deux prémélanges (I) et (II) sont mélangés conjointement jusqu'à ce qu'une émulsion soit formée ;
(d) une amine au moins bifonctionnelle est ensuite versée dans l'émulsion de l'étape (c) ; et
(e) l'émulsion est ensuite chauffée jusqu'à ce que les microcapsules soient formées ; et où le matériau liquide insoluble dans l'eau comprend un pesticide,
où le colloïde protecteur est un copolymère d'alcool polyvinylique ayant des degrés d'hydrolyse de 60 à 99,9 %,
où le copolymère d'alcool polyvinylique contient des comonomères avec des groupes anioniques, et dans lequel l'isocyanate (A) est choisi parmi des isocyanates alicycliques ou aliphatiques.

2. Procédé selon la revendication 1, dans lequel le copolymère d'alcool polyvinylique contient des comonomères avec des groupes anioniques choisis parmi des groupes d'acide carboxyl- et/ou sulfonique.

3. Procédé selon la revendication 1 ou 2, dans lequel le copolymère d'alcool polyvinylique contient 0,1 à 30 % en moles des comonomères avec des groupes anioniques.

4. Procédé selon au moins une des revendications 1 à 3, dans lequel le copolymère d'alcool polyvinylique est utilisé avec des quantités de 0,1 à 20 % en poids, sur la base du poids des microcapsules.

5. Procédé selon au moins l'une des revendications 1 à 4, dans lequel le matériau insoluble dans l'eau comprend un pesticide mélangé avec un solvant huileux.

6. Procédé selon au moins une des revendications 1 à 5, dans lequel l'isocyanate (A) est choisi parmi le diisocyanate d'hexaméthylène ou des diisocyanates de dicyclohexylméthane.

7. Procédé selon au moins une des revendications 1 à 6, dans lequel un mélange d'isocyanate (A) et d'un isocyanate anioniquement modifié (B) est utilisé, dans lequel les diisocyanates anioniquement modifiés (B) sont choisis dans le groupe qui contient au moins un groupe acide sulfonique dans la molécule.

8. Procédé selon la revendication 7, dans lequel le rapport en poids entre les isocyanates (A) et (B) est dans la plage de 10:1 à 1:10.

9. Procédé selon au moins une des revendications 1 à 8, dans lequel l'amine au moins bifonctionnelle utilisée est une polyéthylénimine.

10. Procédé selon au moins une des revendications 1 à 9, dans lequel le pesticide a une solubilité dans l'eau allant jusqu'à 10 g/l à 20 °C.

11. Procédé selon au moins une des revendications 1 à 10, dans lequel le rapport noyau-enveloppe (m/m) des microcapsules est de 20:1 à 1:10.

12. Procédé selon au moins une des revendications 1 à 11, dans lequel les microcapsules ont un diamètre de 1 à 30 µm.

13. Procédé selon au moins une des revendications 1 à 11, dans lequel l'émulsion est ensuite chauffée à au moins 50 °C.

14. Procédé de lutte contre des champignons phytopathogènes et/ou une croissance de plantes indésirables et/ou une attaque par des insectes ou des acariens indésirables et/ou pour réguler la croissance de plantes, dans lequel on laisse agir les microcapsules pouvant être obtenues par un procédé tel que défini dans l'une quelconque des revendications 1 à 13 sur les organismes nuisibles respectifs, leur environnement ou les plantes cultivées devant être protégées contre l'organisme nuisible respectif, sur le sol et/ou sur des plantes indésirables et/ou sur les plantes cultivées et/ou sur leur environnement.
